# EUROPEAN PATENT SPECIFICATION

(11) **EP 4 344 414 B1**
(45) Date of publication and mention of the grant of the patent: **15.04.2026**
(21) Application number: 22757665.9
(22) Date of filing: 04.08.2022
(51) Int. Cl.: A61N 1/36, A61K 39/00, A61N 1/04

(54) **ELECTRODE ASSEMBLY FOR APPLYING TUMOR TREATING FIELDS WITH A SHEET OF ANISOTROPIC MATERIAL**
ELEKTRODENANORDNUNG ZUR ANWENDUNG VON TUMORBEHANDLUNGSFELDERN MIT EINER FOLIE AUS ANISOTROPEM MATERIAL
ENSEMBLE ÉLECTRODE POUR L'APPLICATION DE CHAMPS DE TRAITEMENT DE TUMEURS AVEC UNE FEUILLE DE MATÉRIAU ANISOTROPE

(30) Priority: 06.08.2021 US 202163230438 P; 04.11.2021 US 202163275841 P; 04.11.2021 US 202163275843 P
(43) Date of publication of application: 03.04.2024
(60) Divisional application: 26163081.8
(73) Proprietor: Novocure GmbH, 6340 Baar (CH)
(72) Inventor: WASSERMAN, Yoram, 31905 Haifa (IL); OBUCHOVSKY, Stas, 31905 Haifa (IL); KUPLENNIK, Nataliya, 31905 Haifa (IL); SHAPIRO, David, 31905 Haifa (IL)
(74) Representative: Boden, Keith McMurray
(86) International application number: PCT/IB2022/057233
(87) International publication number: WO 2023/012707

(56) References cited:
- WO-A1-2021/141163
- AU-A1- 2017 279 796
- CN-A- 108 568 029
- US-A1- 2019 224 474
- SONG NA ET AL: "Highly Anisotropic Thermal Conductivity of Layer-by-Layer Assembled Nanofibrillated Cellulose/Graphene Nanosheets Hybrid Films for Thermal Management", APPLIED MATERIALS & INTERFACES, vol. 9, no. 3, 25 January 2017 (2017-01-25), US, pages 2924 - 2932, XP055804316, ISSN: 1944-8244, DOI: 10.1021/acsami.6b11979

## Description

### BACKGROUND

Tumor Treating Fields (TTFields) therapy is a proven approach for treating tumors using alternating electric fields at frequencies between 50 kHz and 1 MHz, such as, for example, 100-500 kHz. The alternating electric fields are induced by electrode assemblies (e.g., arrays of capacitively coupled electrodes, also called transducer arrays) placed on opposite sides of the subject's body. When an AC voltage is applied between opposing electrode assemblies, an AC current is coupled through the electrode assemblies and into the subject's body. And higher currents are strongly correlated with higher efficacy of treatment.

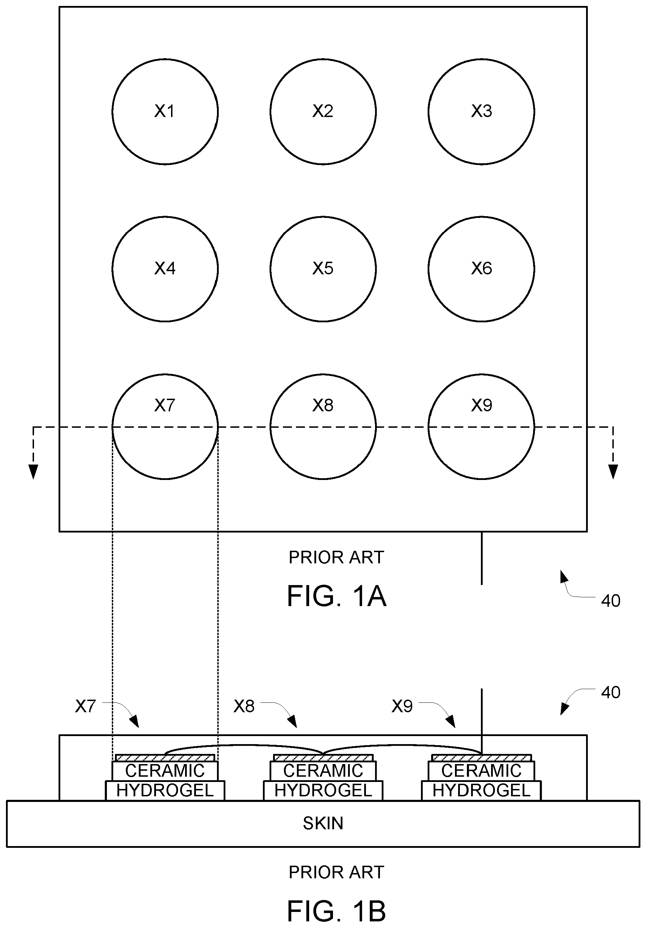

FIG. 1 A is a schematic representation of a prior art electrode assembly 40 including nine prior art electrode elements, labeled X1-X9. FIG. 1B is a cross sectional schematic view of electrode elements X7-X9 of the electrode assembly 40, taken along the dashed line in FIG. 1A.

As shown in FIG. 1B, electrode element X7 (taken as exemplary) includes a metal layer (shown with diagonal hatching) and a ceramic (dielectric) layer. A respective layer of electrically conductive hydrogel is provided between each ceramic layer and the subject's skin, to ensure good electrical contact of the electrode elements with the body. An AC voltage from an AC voltage generator (not shown) is applied to the metal layers of electrode elements in opposing electrode assemblies to generate the TTFields in the subject's body.

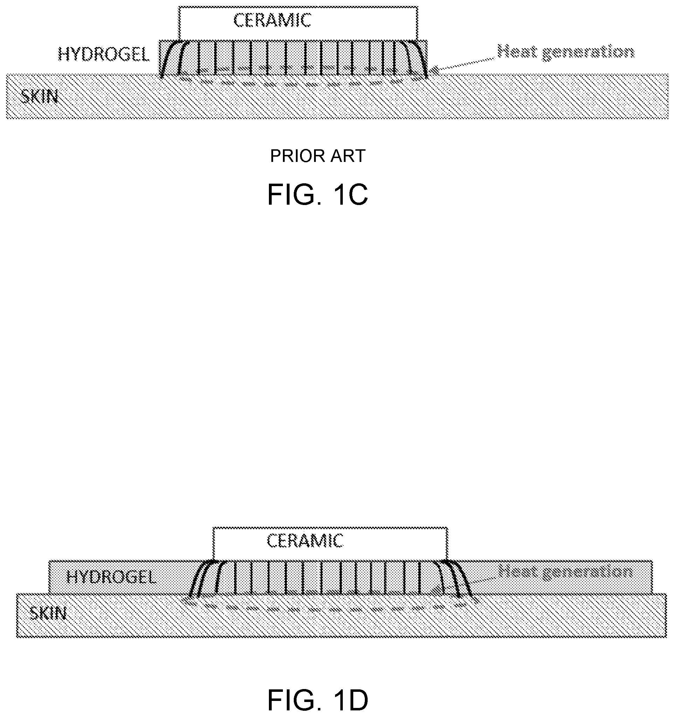

During use, the hydrogel and the skin under the electrode elements heat up, and safety considerations require that the skin temperature remain below a safety threshold (e.g., 41° C). Because the vast majority of the heat appears immediately below the electrode elements X1-X9 (as shown in FIG. 1C), the prior art electrode assembly has hot spots immediately below the electrode elements, and cooler regions positioned between the electrode elements. And those hot spots limit the amount of current that can be delivered through the prior art electrode assemblies.

AU-A-2017279796 discloses an anisotropically electrically conductive material to electrically interface an electrode with a portion of a biological surface, along with methods of forming the anisotropically electrically conductive material. CN 108568029A discloses a graphene-based warm physiotherapy electrode sheet.

### SUMMARY OF THE INVENTION

The invention provides an apparatus for applying an alternating electric field to a subject's body according to claim 1.

Embodiments of the invention are disclosed in the dependent claims. The present disclosure provides also further examples useful for understanding the invention. In particular, the disclosed methods of treatment are not claimed.

### BRIEF DESCRIPTION OF THE DRAWINGS

FIG. 1A is a schematic representation of a prior art electrode assembly.
FIG. 1B is a cross sectional view of electrode elements of the prior art electrode assembly, taken along the dashed line in FIG. 1A.
FIG. 1C is a cross sectional view showing the heat generation properties of a prior art electrode element.
FIG. 1D is a cross sectional view showing the heat generation properties of a hypothetical modification to the FIG. 1B electrode element.
FIG. 2 is a plan schematic representation of an electrode assembly including electrode elements that is used for applying TTFields to a subject's body.
FIG. 3A is a cross sectional representation of a first embodiment including electrode elements E1, E2, taken along the dashed line in FIG. 2.
FIG. 3B is a cross sectional view showing the heat generation properties of the FIG. 3A embodiment.
FIG. 4A is a thermal image of a prior art electrode assembly.
FIG. 4B is a thermal image of an electrode assembly corresponding to the FIG. 3A embodiment.
FIG. 4C is a graph comparing the thermal properties of the prior art electrode assembly with the FIG. 3A embodiment.
FIG. 4D shows a thermal camera image of simulated electrodes arrays built using metal (aluminum) sheets.
FIG. 4E shows a thermal camera image of simulated electrodes arrays built using sheets of anisotropic material (pyrolytic graphite).
FIG. 4F depicts experimental results when electrode arrays with and without a sheet of graphite were used to apply TTFields to the torso of rats.
FIG. 5 is a cross sectional representation of a second embodiment including electrode elements E1, E2, taken along the dashed line in FIG. 2.
FIG. 6 is a cross sectional representation of a third embodiment that includes a single electrode element E1.
FIG. 7 is a cross sectional representation of a fourth embodiment that includes a single electrode element E1.
FIG. 8 is a cross sectional representation of a fifth embodiment that includes a single electrode element E1.
FIG. 9 is a block diagram of a system incorporating two electrode assemblies that is used for applying TTFields to a subject's body.

Various embodiments are described in detail below with reference to the accompanying drawings, wherein like reference numerals represent like elements.

### DESCRIPTION OF THE PREFERRED EMBODIMENTS

This application describes exemplary electrode assemblies that may be used, e.g., for delivering TTFields to a subject's body and treating one or more cancers or tumors located in the subject's body.

When TTFields are applied to a subject's body, the temperature at the subject's body may increase proportionally to the induced electric field. Regulations limit the amount of current that can be driven through a transducer array to an amount that keeps the measured temperature at locations on the subject's body below a temperature threshold. As practiced in the art, the temperature at the location of the transducer arrays on the subject's body is controlled to be below the temperature threshold by reducing the operational current driven by the transducer arrays and reducing the strength of the resulting TTFields. This in turn becomes an over-riding limitation on the TTFields strength that can be used to treat the tumor. Accordingly, there is a need in the art to safely access higher TTField strengths without exceeding the temperature threshold at the subject's skin.

On transducer arrays that comprise multiple electrode elements, the portions of the transducer arrays positioned directly beneath the electrode elements get hotter than the portions of the transducer arrays positioned between the electrode elements. Furthermore, on transducer arrays that comprise multiple electrode elements, higher currents flow through the electrode elements located along the edge of the array compared to the electrode elements located toward the middle of the array. Further still, an electrode element located at a corner or similar sharp bend in the edge of the array will have a higher current than other electrode elements along the edge and near the center of the array. The tendency of a transducer array to drive higher currents through electrode elements located along the edge of the array, and particularly at the corners, is referred to herein as the "edge effect."

An uneven distribution of current through the transducer array due to either the distribution of the electrode elements or the edge effect can lead to higher temperature zones (or "hot spots") e.g., at the corners or edges of the transducer array. These hot spots are the locations that reach the threshold temperature first and therefore control the requirement to reduce the current. As such, the generation of hot spots limits the maximum operational current that may be driven by a transducer array, and the strength of the resulting TTFields.

The inventors have now recognized that a need exists for transducer arrays that reduce or minimize uneven distribution of current and thereby allow the application of higher operating currents. Transducer arrays operated with increased current can induce stronger TTFields in the subject's body, ultimately leading to better patient outcomes. The electrode assemblies disclosed herein allow current and heat to be spread evenly over the array thereby minimizing or eliminating hot spots.

The embodiments described herein incorporate into the electrode assembly a sheet of material having anisotropic thermal properties and/or anisotropic electrical properties, as described below. If the sheet of material has anisotropic thermal properties, then the sheet spreads the heat out more evenly over a larger surface area. If the sheet of material has anisotropic electrical properties, then the sheet spreads the current out more evenly over a larger surface area. In each case, this lowers the temperature of the hot spots and raises the temperature of the cooler regions when a given AC voltage is applied to the electrode assembly (as compared to the prior art configuration described above). Accordingly, the current can be increased (thereby increasing the therapeutic effect) without exceeding the safety temperature threshold at any point on the subject's skin.

In some embodiments, the anisotropic material is anisotropic with respect to electrical conductivity properties. In some embodiments, the anisotropic material is anisotropic with respect to thermal conductivity properties. In some preferred embodiments, the anisotropic material is anisotropic with respect to both electrical conductivity properties and thermal conductivity properties.

The anisotropic thermal properties include directional thermal properties. Specifically, the sheet of anisotropic material has a first thermal conductivity in a direction that is perpendicular to its front face. And the thermal conductivity of the sheet in directions parallel to the front face is more than two times higher than the first thermal conductivity. In some preferred embodiments, the thermal conductivity in the parallel directions is more than ten times higher than the first thermal conductivity. For example, the thermal conductivity of the sheet in directions that are parallel to the front face may be more than: 1.5 times, 2 times, 3 times, 5 times, 10 times, 20 times, 30 times, 100 times, 200 times, or even more than 1,000 times higher than the first thermal conductivity. In some embodiments, the thermal conductivity of the sheet of anisotropic material in directions that are parallel to the front face is between 5 times and 30 times higher than the first thermal conductivity. In some embodiments, the thermal conductivity of the sheet of anisotropic material in directions that are parallel to the front face is between 1.5 times and 10 times higher than the first thermal conductivity. For example, the thermal conductivity of a sheet of pyrolytic graphite in directions that are in the x-y plane is between 10 times and 20 times higher than its thermal conductivity in the perpendicular z-direction.

The anisotropic electrical properties include directional electrical properties. Specifically, the sheet has a first resistance in a direction that is perpendicular to its front face. And resistance of the sheet in directions parallel to the front face is less than the first resistance. In some preferred embodiments, the resistance in the parallel directions is less than half of the first resistance or less than 10% of the first resistance. For example, the resistance of the sheet 70 in directions that are parallel to the front face may be less than: 75%, 50%, 40%, 30%, 20%, 10%, 5%, 1%, 0.5%, 0.1%, or even less than 0.05% of the first resistance. In some embodiments, the resistance of the sheet of anisotropic material in directions that are parallel to the front face is between 10% and 75% of the first resistance. In some embodiments, the resistance of the sheet of anisotropic material in directions that are parallel to the front face is between 0.05% and 10 % of the first resistance. For example, the electrical resistivity of a sheet of pyrolytic graphite in directions that are in the x-y plane is approximately three orders of magnitude (1,000 times) lower than its electrical resistivity in the perpendicular z-direction.

In some embodiments (e.g., when the sheet of anisotropic material is a sheet of pyrolytic graphite), the sheet of anisotropic material has both anisotropic electrical properties and anisotropic thermal properties.

In some embodiments (e.g., when the sheet of anisotropic material is a sheet of pyrolytic graphite), the sheet of anisotropic material is nonmetallic. These embodiments are particularly advantageous in situations where preventing the transfer of ions into a subject's body is desirable. More specifically, using a metallic sheet could result in the transfer of metal ions into a subject's body. In situations where this is not desirable, embodiments that use nonmetallic sheets of anisotropic material are preferable.

The present invention can be understood more readily by reference to the following detailed description, examples, drawings, and claims, and their previous and following description. However, it is to be understood that this invention is not limited to the specific apparatuses, devices, systems, and/or methods disclosed unless otherwise specified, and as such, of course, can vary.

Headings are provided for convenience only and are not to be construed to limit the invention in any manner. Embodiments illustrated under any heading or in any portion of the disclosure may be combined with embodiments illustrated under the same or any other heading or other portion of the disclosure.

Any combination of the elements described herein in all possible variations thereof is encompassed by the invention unless otherwise indicated herein or otherwise clearly contradicted by context.

As used in the specification and the appended claims, the singular forms "a," "an" and "the" include plural referents unless the context clearly dictates otherwise.

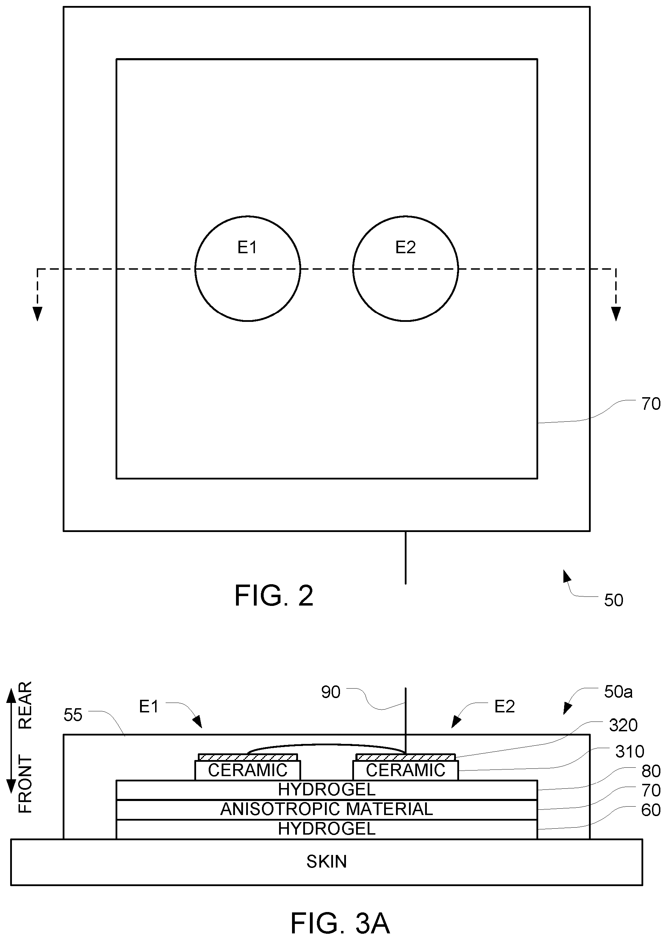

FIG. 2 is a schematic representation of an electrode assembly 50 of an embodiment including electrode elements used for applying TTFields to a subject's body. In FIG. 2, only two electrode elements labeled E1 and E2 are shown, but additional electrode elements may be included in the electrode assembly 50. In alternative embodiments, the electrode assembly 50 includes only a single electrode element. Notably, FIG. 2 depicts an electrode assembly 50 generically, and those electrode assemblies E1 and E2 can have different configurations (e.g., as described below in connection with FIGS. 3A-8).

FIG. 3A is a cross sectional representation of a first embodiment of an electrode assembly 50a including electrode elements E1, E2, taken along the dashed line in FIG. 2.

In the FIG. 3A embodiment, the electrode assembly 50a includes a sheet of anisotropic material 70 having a front face (facing towards the subject's skin in FIG. 3A) and a rear face. This sheet 70 has a first thermal conductivity in a direction that is perpendicular to the front face. Thermal conductivity of the sheet 70 in directions that are parallel to the front face is more than two times higher than the first thermal conductivity. In some preferred embodiments, the thermal conductivity of the sheet 70 in directions that are parallel to the front face is more than ten times higher than the first thermal conductivity. The sheet 70 in the FIG. 3A embodiment is also anisotropic in another respect. More specifically, the sheet 70 has a first resistance in a direction that is perpendicular to the front face, and the resistance of the sheet in directions that are parallel to the front face is less than half of the first resistance. In some embodiments, the resistance of the sheet in directions that are parallel to the front face is less than 10% of the first resistance.

The electrode assembly 50a includes a sheet of conductive anisotropic material 70 having a front face (facing towards the subject's skin in FIG. 3A) and a rear face. The sheet of conductive anisotropic material 70 could be a sheet of graphite. Examples of suitable forms of graphite include synthetic graphite, such as pyrolytic graphite (including, but not limited to, Pyrolytic Graphite Sheet (PGS), available from Panasonic Industry, Kadoma, Osaka, Japan), other forms of synthetic graphite, including but not limited to, graphite foil made from compressed high purity exfoliated mineral graphite (including, but not limited to, that supplied by MinGraph^{®} 2010A Flexible Graphite, available from Mineral Seal Corp., Tucson, Arizona, USA), or graphitized polymer film, e.g., graphitized polyimide film, (including, but not limited to, that supplied by Kaneka Corp., Moka, Tochigi, Japan. In alternative embodiments, conductive anisotropic materials other than graphite may be used instead of graphite.

In some embodiments, the sheet of anisotropic material 70 is a sheet of pyrolytic graphite. Thermal conductivity of pyrolytic graphite sheets in directions that are parallel to the front face of those sheets (i.e., in the x-y plane) is typically more than 50 times higher than the thermal conductivity of those sheets in directions that are perpendicular to the front face (i.e., in the z direction). And electrical resistivity of pyrolytic graphite sheets in directions that are parallel to the front face of those sheets (i.e., in the x-y plane) is typically less than 2% of the electrical resistivity of those sheets in directions that are perpendicular to the front face (i.e., in the z direction).

In other embodiments, the sheet of anisotropic material 70 is graphite foil made from compressed high purity exfoliated mineral graphite. In other embodiments, the anisotropic material may be pyrolytic carbon, or graphitized polymer film. In other embodiments, the anisotropic material may be boron nitride. Other embodiments may utilize sheets of other conducting materials with anisotropic properties. In some embodiments (e.g., when the sheet of anisotropic material is a sheet of a synthetic graphite, such as pyrolytic graphite or the compressed high purity exfoliated mineral graphite or graphitized polymer film), the sheet of anisotropic material 70 is nonmetallic.

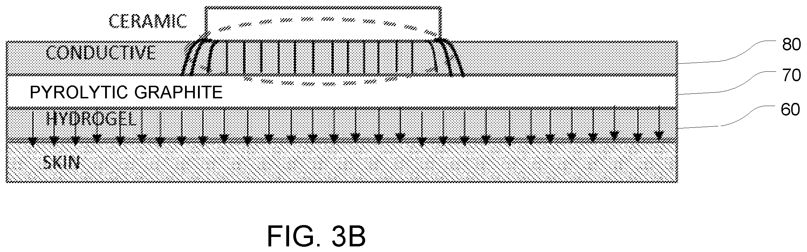

The electrode assembly 50a further includes at least one layer of conductive material 60 disposed on the front face of the sheet 70, and the at least one layer of conductive material 60 has a biocompatible front surface. Note that the embodiment illustrated in FIG. 3, there is only a single layer of conductive material 60, and that single layer is biocompatible. But in alternative embodiments (not shown) there could be more than one layer, in which case only the front layer must be biocompatible. The at least one layer of material 60 is configured to ensure good electrical contact between the device and the body. In some embodiments, the at least one layer of material 60 should cover the entire front face of the sheet of anisotropic material 70. The at least one layer of material 60 may be the same size or larger than the sheet of anisotropic material 70. In some embodiments (and as shown in FIG. 3A), the at least one layer of conductive material 60 comprises a single layer of hydrogel. In these embodiments, the hydrogel may have a thickness between 50 and 2000 µm, such as, from 100 to 1000 µm, or even 300 to 500 µm. In some embodiments, the at least one layer of conductive material 60 is a single layer of non-hydrogel biocompatible conductive adhesive. In some embodiments, the at least one layer of conductive material 60 is a single layer of non-hydrogel biocompatible conductive adhesive such as the developmental product FLX068983 - FLEXcon^{®} OMNI-WAVE^{™} TT 200 BLACK H-502 150 POLY H-9 44PP-8 from FLEXcon, Spencer, MA, USA, or other such OMNI-WAVE products from FLEXcon; or ARcare^{®} 8006 electrically conductive adhesive composition manufactured and sold by Adhesives Research, Inc. (Glen Rock, PA, USA). Non-hydrogel conductive adhesives may comprise a waterless polymer with adhesive properties and carbon particles, powder, fibers, flakes or nanotubes. The adhesive polymer may be, for example, an acrylic polymer or a silicone polymer, or combination thereof, which may be available as acrylic- or silicone-based carbon-filled adhesive tapes. The adhesive may additionally include one or more conductive polymer (such as, for example, polyaniline (PANI), or poly(3,4-ethylenedioxythiophene (PEDOT), or others known in the art). The conductive filler in the at least one layer of conductive material 60 should be non-metallic. In these embodiments, the biocompatible conductive adhesive may have a thickness between 10 and 2,000 µm, such as, from 20 to 1000 µm, or even 30 to 400 µm.

The electrode assembly 50a further includes a first electrode element E1 positioned behind the sheet 70. The first electrode element E1 has a first front face disposed in electrical contact with the rear face of the sheet 70. In the FIG. 3A embodiment, the first electrode element E1 includes a first layer of dielectric (e.g., ceramic) material 310 having a front face and a rear face, and a first layer of metal 320 disposed on the rear face of the first layer of dielectric material 310. The front face of the first layer of dielectric material 310 is the first front face of the first electrode element E1. Note that while the figures (e.g., FIG. 3A) depict the dielectric material 310 as "ceramic," a variety of other suitable dielectric materials may be used instead of ceramic materials. Examples include a polymer layer that has a dielectric constant of at least 10, or another material having a dielectric constant of at least 10.

In some embodiments, the layer of dielectric material 310 can have a dielectric constant ranging from 10 to 50,000. In some embodiments, the layer of dielectric material 310 comprises a high dielectric polymer material such as poly(vinylidene fluoride-trifluoroethylene-chlorotrifluoroethylene) and/or poly(vinylidene fluoride-trifluoroethylene-1-chlorofluoroethylene). Those two polymers are abbreviated herein as "Poly(VDF-TrFE-CTFE)" and "Poly(VDF-TrFE-CFE)," respectively. These embodiments are particularly advantageous because the dielectric constant of these materials is on the order of 40. In some embodiments, the polymer layer can be poly(vinylidene fluoride-trifluoroethylene-chlorotrifluoroethylene-chlorofluoroethylene) or "Poly(VDF-TrFE-CTFE-CFE)."

In some embodiments, the layer of dielectric material 310 comprises a terpolymer comprising polymerized units of monomers such as VDF, TrFE, CFE and/or CTFE in any suitable molar ratio. Suitable terpolymers include those, for example, having 30 to 80 mol% VDF, 5 to 60 mol% TrFE, with CFE and/or CTFE constituting the balance of the mol% of the terpolymer.

In some embodiments, the sheet 70 has a centroid, and the centroid of the first front face of the first electrode element E1 is positioned less than 3 cm away from the centroid of the sheet 70. In some embodiments, the sheet 70 has a centroid and a dimension parallel to the rear face of the sheet 70 (e.g., a length or a width), and the centroid of the first front face of the first electrode element E1 is positioned away from the centroid of the sheet 70 by less than 30%, or by less than 10% of the dimension.

The electrode assembly 50a further includes a first rear layer of conductive material 80 positioned between the first front face of the first electrode element E1 (i.e., the front face of the first layer of dielectric material 310) and the rear face of the sheet 70. The first rear layer of conductive material 80 facilitates the electrical contact between the first front face of the first electrode element E1 and the rear face of the sheet 70. In the illustrated embodiment, the rear layer of conductive material 80 is a layer of hydrogel. But in alternative embodiments, a different conductive material (e.g., conductive grease, conductive adhesive, conductive tape, conductive composite, etc.) could be used. In some embodiments, the conductive material 80 may be a non-hydrogel conductive adhesive, such as described above.

The electrode assembly 50a may optionally include one or more additional electrode elements. In the illustrated embodiment, the electrode assembly 50a includes a second electrode element E2 positioned behind the sheet 70. The second electrode element E2 has a second front face disposed in electrical contact with the rear face of the sheet 70. The two electrode elements E1, E2 in FIG. 3A have identical structures. Thus, the second electrode element E2 includes a second layer of dielectric (e.g., ceramic) material 310 having a front face and a rear face, and a second layer of metal 320 disposed on the rear face of the second layer of dielectric material 310. The front face of the second layer of dielectric material 310 is the second front face of the second electrode element E2. In some embodiments, the collective area of all the electrode elements is less than the area of the sheet 70, less than half the area of the sheet 70, less than one quarter the area of the sheet 70, or less than one tenth the area of the sheet 70.

The first rear layer of conductive material 80 is positioned between the second front face of the second electrode element E2 (i.e., the front face of the second layer of dielectric material 310) and the rear face of the sheet 70. The first rear layer of conductive material 80 facilitates the electrical contact between the second front face of the second electrode element E2 and the rear face of the sheet 70. As described for E1, and as shown in FIG. 3A, the conductive material 80 may be a layer of hydrogel, but in alternative embodiments, a different conductive material may be used (e.g., conductive grease, conductive adhesive including the non-hydrogel conductive adhesive described above, conductive tape, conductive composite, etc.).

The metal layers 320 of all of the electrode elements (i.e., E1 and E2 in the illustrated embodiment), may be wired together (e.g., using wires, traces on a flex circuit, etc.) to a lead 90. The lead 90 supplies an AC voltage from an AC voltage generator (not shown) to the electrode elements to generate the TTFields when the electrode assembly 50a is affixed to the subject's body for treatment.

Optionally, the electrode assembly 50a includes a flexible self-adhesive backing 55 configured to support the sheet 70, the first electrode element E1 (and any other electrode elements present in the electrode assembly), and the at least one layer of conductive material 60 so that the front surface of the at least one layer of conductive material 60 can be positioned against the subject's skin.

As noted above, FIG. 2 is a plan schematic representation of an electrode assembly 50 including electrode elements E1, E2. This view of FIG. 2 (not to scale) also demonstrates that the area of the sheet 70 is larger (e.g., at least 2 times larger, at least 4 times larger, or at least 10 times larger) than the combined areas of the electrode elements E1, E2. When an AC voltage is applied to the electrode elements E1, E2, heat spreads out across the entire sheet 70, which minimizes or eliminates hot spots. In addition to spreading and altering the directionality of the heat (minimizing heat in specific locations), the spread has an additional effect in dissipating the heat since the majority is directed in the x-y plane to the edges of the sheet, which terminate in a room temperature (cooler) heat sink, rather than proceeding in the z-direction to a much warmer body temperature. The area therefore cools much faster.

This reduction in hot spots (as compared to the prior art) becomes apparent by comparing FIG. 1C to FIG. 3B. More specifically, FIG. 1C shows the current distribution and heat generation for prior art electrode elements, each of which is positioned on a conductive hydrogel layer that covers about the same area (in the x-y plane) as the electrode element. As shown in FIG. 1C, all the current passes through the hydrogel layer directly beneath the electrode elements, which results in hot spots directly beneath the electrode elements.

One might initially think that this problem could be solved by increasing the area of the hydrogel to cover all the regions between the electrode elements (i.e., by covering a significantly larger area in the x-y plane than that of the electrode elements). But this is not the case. More specifically, FIG. 1D shows the current distribution and heat generation for this hypothetical electrode assembly. As shown in FIG. 1D, all the current still passes through the hydrogel layer directly beneath the electrode elements, which results in hot spots directly beneath the electrode elements.

In contrast, FIG. 3B shows the current distribution for the FIG. 3A embodiment. As shown in FIG. 3B, the current is still distributed in the rear conductive material layer (for example, 80 in FIG. 3B) only in the area below the electrode element. However, the sheet of anisotropic material 70 spreads the heat out across its entire area because the thermal conductivity in the horizontal directions (i.e., in directions parallel to the face of the sheet) is much higher than its thermal conductivity in the vertical direction. In addition to spreading out the heat, the low electrical resistance of the sheet 70 in the horizontal direction spreads the current outward throughout the sheet 70, and this spread-out current distribution continues in the layer of conductive material 60 (hydrogel in both FIG. 3A and FIG. 3B), and thence to the subject's skin. Because the current and heat in this embodiment are both spread out over a larger area of the layer of conductive material 60, hotspots are eliminated (or at least minimized). This means that for a given applied AC voltage, the hottest point beneath the electrode assembly in the FIG. 3A/B embodiment will have a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 3A embodiment. And this increase in current will advantageously increase the efficacy of the TTFields treatment. Similar results can be achieved when the hydrogel is replaced with a conductive adhesive composite.

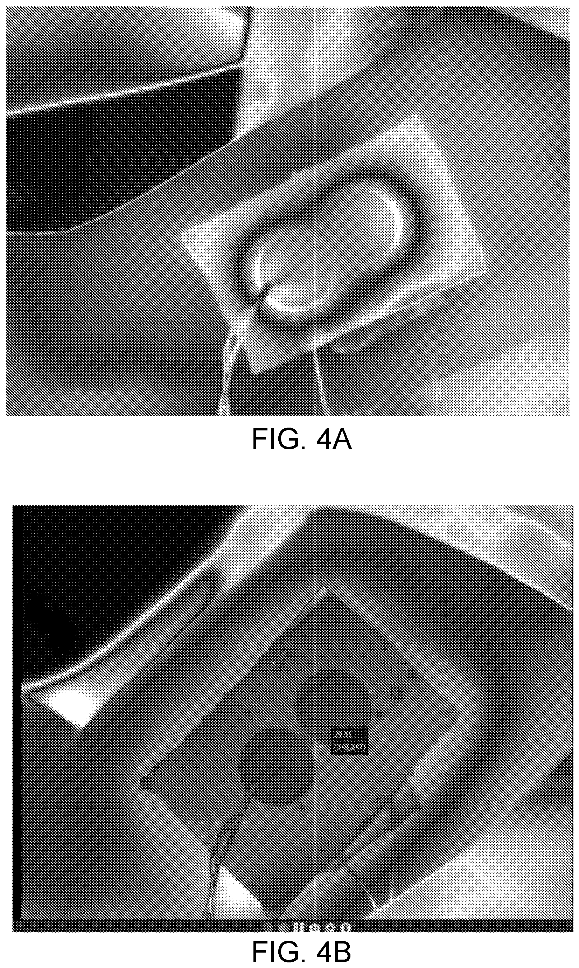

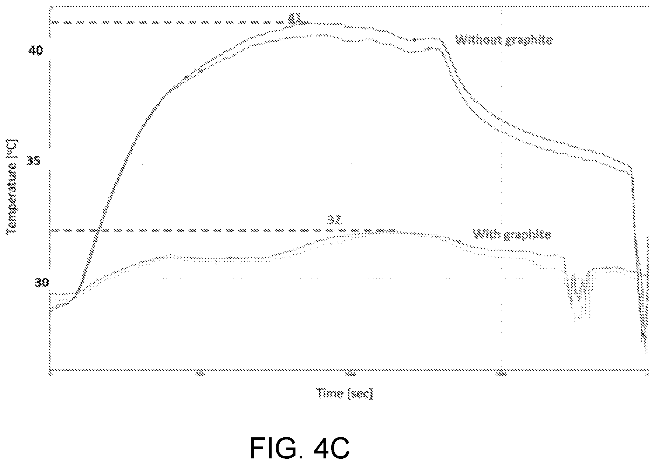

The superior performance of the FIG. 3A embodiment is demonstrated in FIGS. 4A, 4B, and 4C. FIG. 4A is a thermal image of a prior art electrode assembly that includes two electrode elements and a layer of hydrogel disposed on the front faces of the electrode elements (see, e.g., FIG. 1B). There is no sheet of anisotropic material between the front faces of the electrode elements and the rear face of the layer of hydrogel. In use, the front face of the layer of hydrogel is positioned on the subject's skin. FIG. 4A shows hot spots generated in the areas that correspond to the electrode elements.

FIG. 4B is a thermal image of an electrode assembly corresponding to the FIG. 3A embodiment where pyrolytic graphite was used as the anisotropic material. FIG. 4B shows that hot spots such as those generated in the prior art electrode assembly have been minimized, and also that the maximum temperature has been reduced. FIG. 4C is a graph comparing the thermal performance of the FIG. 3A embodiment (utilizing pyrolytic graphite as the anisotropic material) with the prior art (no anisotropic material) for the same applied current (500 mA). Notably, the hottest portion of the prior art electrode assembly was 41° C. But when the same 500 mA current was applied to the FIG. 3A embodiment, the hottest portion of the electrode assembly was only 32° C. Similar experiments were performed utilizing graphite foil made from compressed high purity exfoliated mineral graphite as the anisotropic material, with similar results.

In a related experiment, optimized conventional arrays (no anisotropic material), running with 2 A applied current, ran up to the maximum 40 °C average temperature, and were thereby limited. The same type of array with an added pyrolytic graphite sheet (in the manner of the FIG. 3A embodiment) was able to run at an increased power level (with 3 A applied current), and ran at 38 °C average temperature, 2-3 °C below the temperature threshold limit. This result suggests that the inventive apparatus and methods described herein should be able to achieve more beneficial treatment results by operating at higher applied currents.

An experimental simulation of electrodes for treating a target location in a body compared the heat distribution obtained using pyrolytic graphite sheets to the heat distribution obtained using metal sheets. In one half of the experiment, a phantom gel was placed sandwiched between two sheets of metal (aluminum) and a voltage was applied (directly to the center of the sheet) between the two metal sheets. In the other half of the experiment, a phantom gel was placed sandwiched between two sheets of pyrolytic graphite and a voltage was applied (directly to the center of the sheet) between the two pyrolytic graphite sheets. Metals are generally excellent conductors (both thermal conductivity and electrical conductivity). Pyrolytic graphite sheets also have good electrical conductivity (and higher electrical conductivity in the x-y plane than in the perpendicular z-direction). However, the electrical conductivity in x-y plane for pyrolytic graphite is two orders of magnitude smaller than that of the conventional metals. When voltage is applied between a pair of metal sheets (aluminum), the high conductivity and equipotential properties of aluminum sheet result in a higher current density at the edges of the sheet resulting in an unequal heating of different areas. In contrast, applying voltage between two graphite sheets beneficially results in much more uniform current densities at the sheet center and edges (due to the higher resistance in the x-y plane compared to metal), and results in a more uniform temperature profile of the sheet.

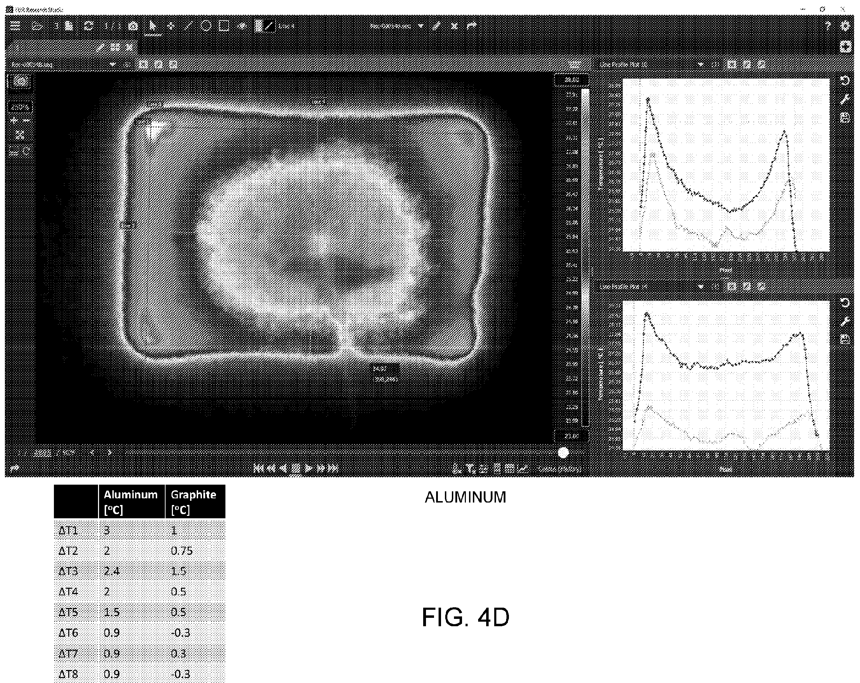

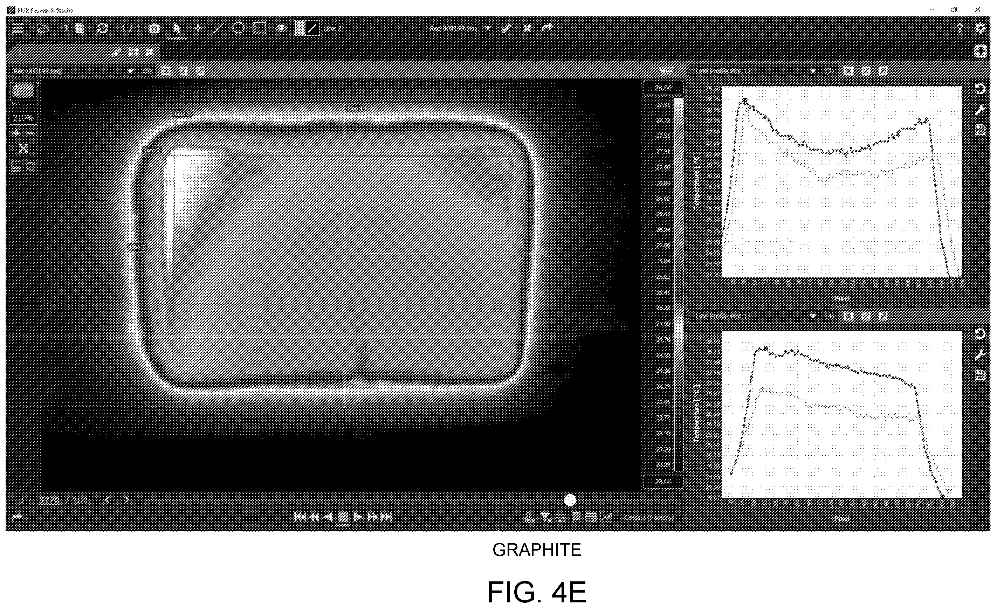

FIGS. 4D and 4E respectively show thermal camera images of the simulated electrodes arrays built using metal (aluminum) sheets and the simulated electrode arrays built using sheets of anisotropic material (pyrolytic graphite). The aluminum sheet results in an uneven heat distribution map which causes the outer edges to reach the threshold temperature first and therefore control the requirement to reduce the current. In contrast, the sheet of pyrolytic graphite produces a very even heat distribution across the entire sheet.

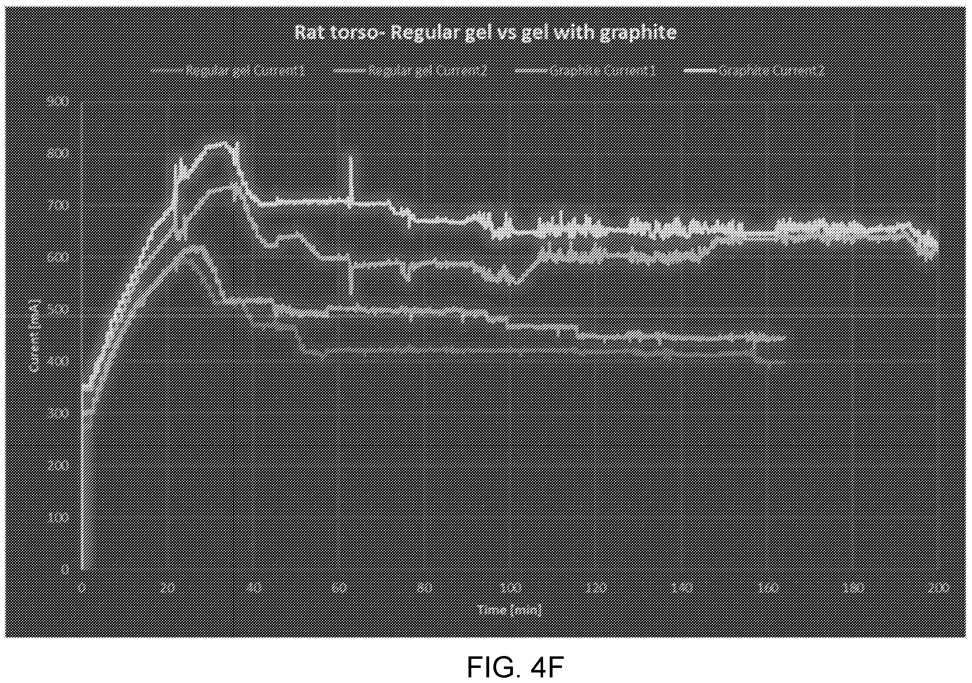

FIG. 4F depicts experimental results when electrode arrays with and without a sheet of graphite were used to apply TTFields to the torso of rats (using small animal arrays). The two lower traces show the measured current for two rats when the prior art electrode arrays depicted in FIG. 1A/1B were used, while the two upper traces show the measured current for two rats when the electrode elements depicted in FIG. 3A were used (using a sheet of graphite as the layer of anisotropic material). The thermal setpoint was identical for all runs. Notably, when the sheet of graphite was included, the improved heat and current distribution attributable to the graphite resulted in resistances that were 20% lower and currents that were 50% higher for the same thermal setpoint. And because higher currents are associated with improved outcomes, these experiments show that incorporating a layer of anisotropic material into the electrode arrays can provide improved outcomes.

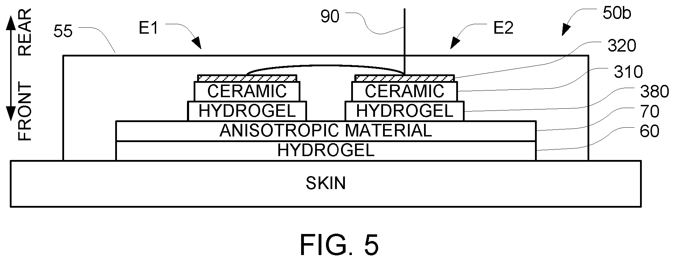

FIG. 5 is a cross sectional representation of a second embodiment of an electrode assembly 50b including electrode elements E1, E2, taken along the dashed line in FIG. 2. The FIG. 5 embodiment is similar to the FIG. 3A embodiment in all respects (including the figure labeling) except as follows. The FIG. 3A embodiment includes a large rear layer of conductive material 80 (e.g., hydrogel) positioned between the sheet 70 and the front faces of both the first and second electrode elements E1 and E2. In contrast, the FIG. 5 embodiment includes a separate region of conductive material 380 for each individual electrode element. Thus, the FIG. 5 embodiment includes a first rear layer of conductive material 380 positioned between the first front face of the first electrode element E1 and the rear face of the sheet 70, and also includes a second rear layer of conductive material 380 positioned between the second front face of the second electrode element E2 and the rear face of the sheet 70. The first and second rear layers of conductive material 380 facilitate the electrical contact between the respective electrode front faces and the rear face of the sheet 70. In the illustrated embodiment, the rear layers of conductive material 380 are layers of hydrogel. But in alternative embodiments, different conductive materials (e.g., conductive grease, conductive adhesive including the non-hydrogel conductive adhesives discussed above, conductive tape, conductive composite, etc.) could be used. In some embodiments, the collective area of all the electrode elements is less than the area of the sheet 70, less than half the area of the sheet 70, less than one quarter the area of the sheet 70, or less than one tenth the area of the sheet 70.

As in the FIG. 3A embodiment, the current in the FIG. 5 embodiment is still concentrated in the rear layers of conductive material 380 only in the areas below the electrode elements. The sheet of anisotropic material 70 spreads out the heat and the current as described above in connection with the FIG. 3A embodiment, which eliminates or at least minimizes hot spots. This means that for a given applied AC voltage, the hottest point beneath the electrode assembly in the FIG. 5 embodiment will be at a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 5 embodiment. And this increase in current will advantageously increase the efficacy of the TTFields treatment.

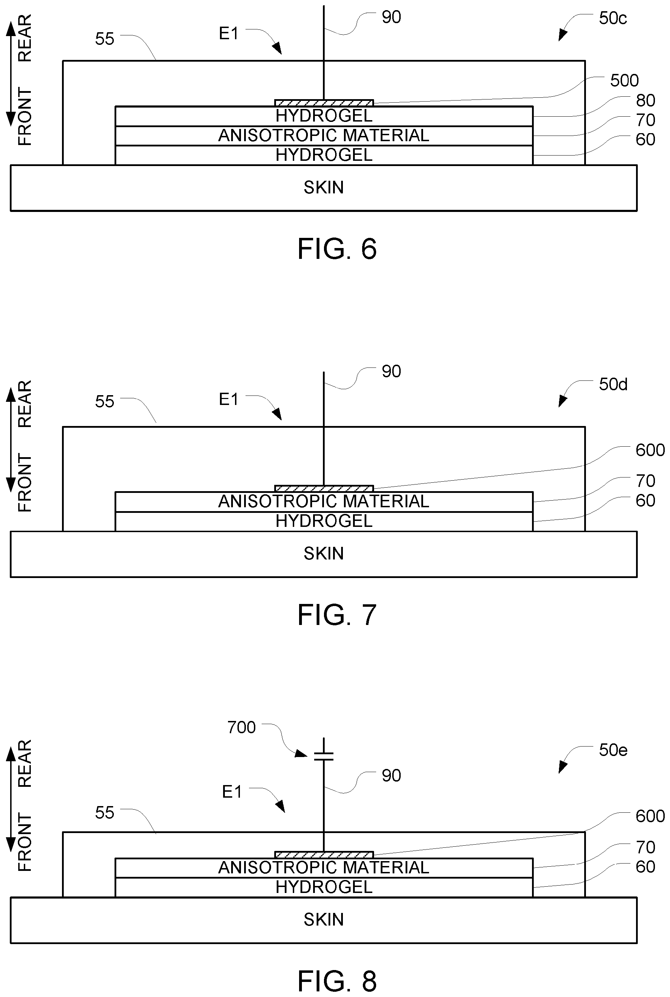

FIG. 6 is a cross sectional representation of a third embodiment of an electrode assembly 50c that includes a single electrode element E1. The embodiment of FIG. 6 is similar to the embodiment of FIG. 3A except the FIG. 6 embodiment does not include the layer of dielectric material. In the FIG. 6 embodiment, the electrode assembly 50c includes a sheet of anisotropic material 70 having a front face (facing towards the subject's skin in FIG. 6) and a rear face. This sheet 70 is similar to the sheet 70 described above in connection with FIG. 3A. In some embodiments, the sheet of anisotropic material 70 is a sheet of synthetic graphite. In some embodiments, the sheet of anisotropic material 70 is a sheet of pyrolytic graphite. In other embodiments, the sheet of anisotropic material 70 is graphite foil made from compressed high purity exfoliated mineral graphite (e.g., MinGraph^{®} 2010A Flexible Graphite). In other embodiments, the sheet of anisotropic material 70 is a sheet of graphitized polymer film. In other embodiments, the sheet of anisotropic material 70 is a sheet of pyrolytic carbon. In other embodiments, the anisotropic material may be boron nitride. In other embodiments, the sheet of anisotropic material 70 is a sheet of another conductive anisotropic material. In some embodiments (e.g., when the sheet of anisotropic material is a sheet of a synthetic graphite, such as pyrolytic graphite or the compressed high purity exfoliated mineral graphite or graphitized polymer film), the sheet of anisotropic material 70 is nonmetallic.

The electrode assembly 50c further includes at least one layer of biocompatible conductive material 60 disposed on the front face of the sheet 70, and the at least one layer of conductive material 60 has a biocompatible front surface. Note that the embodiment illustrated in FIG. 6, there is only a single layer of conductive material 60, and that single layer is biocompatible. But in alternative embodiments (not shown) there could be more than one layer, in which case only the front layer must be biocompatible. The at least one layer of conductive material 60 is configured to ensure good electrical contact between the device and the body. In a preferred embodiment, the at least one layer of conductive material 60 should cover the entire front face of the sheet of anisotropic material 70. The at least one layer of conductive material 60 may be the same size or larger (i.e., cover the same area or larger) than the sheet of anisotropic material 70. In some embodiments, the at least one layer of conductive material 60 comprises a single layer of hydrogel. In these embodiments, the hydrogel may have a thickness between 50 and 2000 µm, such as, from 100 to 1000 µm, or even 300 to 500 µm. In some embodiments, the at least one layer of conductive material 60 is a single layer of non-hydrogel biocompatible conductive adhesive as discussed above. In some embodiments, the at least one layer of conductive material 60 is a single layer of non-hydrogel biocompatible conductive adhesive such as the OMNI-WAVE products from FLEXcon, or the ARcare^{®} products from Adhesives Research, Inc., discussed above. Non-hydrogel conductive adhesives may comprise a waterless polymer with adhesive properties (for example, an acrylic polymer or a silicone polymer, or combination thereof) and a conductive filler. The conductive filler in the at least one layer of conductive material 60 should be non-metallic. In these embodiments, the biocompatible conductive adhesive may have a thickness between 10 and 2,000 µm, such as, from 20 to 1000 µm, or even 30 to 400 µm.

The electrode assembly 50c further includes a first electrode element E1 positioned behind the sheet 70. The first electrode element E1 includes a piece of metal 500 having a front face disposed in electrical contact with the rear face of the sheet 70. In the FIG. 6 embodiment, the front face of the piece of metal 500 is the first front face of the first electrode element E1. Accordingly, the FIG. 6 embodiment differs from the FIG. 3A or FIG. 5 embodiments by lacking a layer of dielectric material. The positional relationship between the first electrode element E1 and the sheet 70 in this FIG. 6 embodiment may be as described above in connection with FIG. 3A.

The electrode assembly 50c further includes a first rear layer of conductive material 80 positioned between the first front face of the first electrode element E1 (i.e., the front face of the piece of metal 500) and the rear face of the sheet 70. The first rear layer of conductive material 80 facilitates the electrical contact between the first front face of the first electrode element E1 and the rear face of the sheet 70. In the illustrated embodiment, the rear layer of conductive material 80 is a layer of hydrogel. But in alternative embodiments, a different conductive material (e.g., conductive grease, conductive adhesive including the non-hydrogel conductive adhesive described above, conductive tape, conductive composite, etc.) could be used.

The piece of metal 500 of the electrode element E1 is wired (e.g., using wires, traces on a flex circuit, etc.) to a lead 90, which supplies an AC voltage from an AC voltage generator (not shown) to the electrode element to generate the TTFields when the electrode assembly 50c is affixed to the subject's body for treatment.

The electrode assembly 50c may optionally include one or more additional electrode elements (not shown) having a structure identical to electrode element E1 and positioned to have the same functionality. In such case, the pieces of metal 500 of all the electrode elements may be wired together (e.g., using wires, traces on a flex circuit, etc.) to the lead 90.

In some embodiments that include only a single electrode element E1, the area of the sheet 70 is larger (e.g., at least 2 times larger, at least 4 times larger, or at least 10 times larger) than the area of the electrode element E1. In some embodiments that include a plurality of electrode elements (not shown) the area of the sheet 70 is larger (e.g., at least 2, 4, or 10 times larger) than the collective area of all of the electrode elements. When an AC voltage is applied to the electrode elements, the heat spreads out across the entire sheet 70, which minimizes or eliminates hot spots.

Similar to the FIG. 3A embodiment, the sheet of anisotropic material 70 in the FIG. 6 embodiment spreads out the heat and the current as described above in connection with the FIG. 3A embodiment, which eliminates or at least minimizes hot spots. This means that for a given applied AC voltage, the hottest point beneath the electrode assembly in the FIG. 6 embodiment will have a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 6 embodiment. And this increase in current will advantageously increase the efficacy of the TTFields treatment.

FIG. 7 is a cross sectional representation of a fourth embodiment of an electrode assembly 50d that includes a single electrode element E1. The FIG. 7 embodiment is similar to the FIG. 6 embodiment except that the first front face of the first electrode element E1 (i.e., the front face of the piece of metal 600) is positioned in direct contact with the rear face of the sheet 70 (instead of being electrically connected via an intervening layer of conductive material).

Similar to the FIG. 6 embodiment, the sheet of anisotropic material 70 in the FIG. 7 embodiment spreads out the heat and the current as described above in connection with the FIG. 3A embodiment, which eliminates or at least minimizes hot spots. This means that for a given applied AC voltage, the hottest point beneath the electrode assembly in the FIG. 7 embodiment will have a lower temperature than the hottest point beneath the electrode assembly in the FIG. 1 prior art example. Accordingly, the current can be increased (with respect to the prior art current) without exceeding the safety temperature threshold at any point beneath the electrode assembly in the FIG. 7 embodiment. And this increase in current will advantageously increase the efficacy of the TTFields treatment.

FIG. 8 is a cross sectional representation of a fifth embodiment of an electrode assembly 50e that includes a single electrode element E1. The FIG. 8 embodiment is similar to the FIG. 7 embodiment, but it adds a capacitor 700 connected in series with and behind the piece of metal 600. A similar addition of a capacitor 700 connected in series with and behind the piece of metal 600 could also be envisioned for the FIG. 6 embodiment.

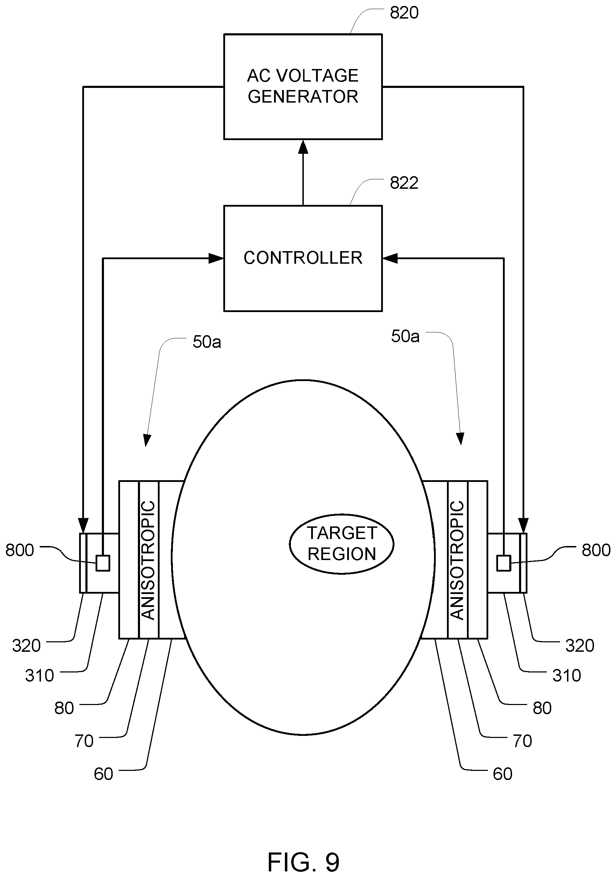

FIG. 9 shows how a pair of the FIG. 3A electrode assemblies 50a may be used to apply an alternating electric field to a target region in the subject's body. The subject could be a human or another mammal, including but not limited to rats and mice. (Note that any of the electrode assemblies described above in connection with FIGS. 5-8 may be used instead of the FIG. 3A electrode assemblies 50a shown here).

The method includes positioning a first electrode assembly 50a at a first position on or in the subject's body. (In the example depicted in FIG. 9, the first electrode assembly 50a is positioned on the subject's skin at the right of the subject's head facing a target region, e.g., a tumor.) The first electrode assembly 50a may be constructed as described earlier herein. In the FIG. 9 embodiment, the first electrode assembly 50a includes a first sheet 70 of anisotropic material 70 having a first front face and a first rear face. The first sheet 70 has a first thermal conductivity in a direction that is perpendicular to the first front face. Thermal conductivity of the first sheet 70 in directions that are parallel to the first front face of the sheet 70 is more than two times higher than the first thermal conductivity. The first sheet 70 has a first resistance in a direction that is perpendicular to the front face, and the resistance of the first sheet in directions that are parallel to the front face is less than half of the first resistance. During use, the first electrode assembly 50a is positioned so that the first front face of the first sheet 70 faces the target region.

The method also includes positioning a second electrode assembly 50a at a second position in or on the subject's body. (In the example depicted in FIG. 9, the second electrode assembly 50a is positioned on the subject's skin at the left of the subject's head facing the target region.) The second electrode assembly 50a may be constructed as described earlier herein. In the FIG. 9 embodiment, the second electrode assembly 50a includes a second sheet 70 of anisotropic material 70 having a second front face and a second rear face. The second sheet 70 has a second thermal conductivity in a direction that is perpendicular to the second front face. Thermal conductivity of the second sheet 70 in directions that are parallel to the second front face of the sheet 70 is more than two times higher than the second thermal conductivity. The second sheet 70 has a second resistance in a direction that is perpendicular to the front face, and the resistance of the second sheet in directions that are parallel to the front face is less than half of the second resistance. During use, the second electrode assembly 50a is positioned so that the second front face of the second sheet 70 faces the target region.

The method further includes applying an alternating voltage between the first electrode assembly 50a and the second electrode assembly 50a. The applying is performed after positioning the first electrode assembly 50a and the second electrode assembly 50a. The applying may be implemented by applying the alternating voltage between (i) a first electrode element disposed in electrical contact with the first rear face of the first sheet 70 and (ii) a second electrode element disposed in electrical contact with the second rear face of the second sheet 70.

In some embodiments, the first electrode assembly 50a further includes a first layer of biocompatible conductive material 60 disposed on the first front face of the first sheet 70. Correspondingly, the second electrode assembly further includes a second layer of biocompatible conductive material 60 disposed on the second front face of the second sheet 70. As described above, the biocompatible conductive material 60 may be hydrogel or may be a conductive grease, conductive adhesive including the non-hydrogel conductive adhesives discussed above, conductive tape, conductive composite, etc.

In some embodiments, the first electrode assembly 50a further includes a first rear layer of conductive material 80 (as described above) positioned between the first front face of the first electrode element of the first electrode assembly 50a and the first rear face of the first sheet 70. Correspondingly, the second electrode assembly further includes a second rear layer of conductive material 80 (as described above) positioned between the second front face of the second electrode element of the second electrode assembly and the second rear face of the second sheet 70.

In some embodiments, each of the first and second sheets of anisotropic material 70 is a sheet of synthetic graphite. In some embodiments, the sheet of anisotropic material 70 is a sheet of pyrolytic graphite. In other embodiments, each of the first and second sheets of anisotropic material 70 is graphite foil made from compressed high purity exfoliated mineral graphite (e.g., MinGraph^{®} 2010A Flexible Graphite, available from Mineral Seal Corp., Tucson Arizona). In other embodiments, the sheet of anisotropic material 70 is a sheet of graphitized polymer film. In other embodiments, the anisotropic material may be pyrolytic carbon. In other embodiments, the anisotropic material may be boron nitride. Other embodiments may utilize sheets of other conducting materials with anisotropic properties. In some embodiments (e.g., when the sheets of anisotropic material are sheets of synthetic graphite, such as pyrolytic graphite or compressed high purity exfoliated mineral graphite or graphitized polymer film), the sheets of anisotropic material 70 are nonmetallic.

The alternating voltage between the first electrode assembly and the second electrode assembly may be applied by an AC voltage generator 820. In some embodiments, the frequency of the alternating voltage is between 50 kHz and 1 MHz, or between 100 kHz and 500 kHz. In the illustrated example, the AC voltage generator is controlled by a controller 822. The controller 822 may use temperature measurements to control the amplitude of the current to be delivered via the first and second electrode assemblies 50a in order to maintain temperatures below a safety threshold (e.g., 41° C). This may be accomplished, for example, by measuring a first temperature of the first electrode element, measuring a second temperature of the second electrode element, and controlling the applying of the alternating voltage based on the first temperature and the second temperature, as described below.

FIG. 9 depicts one example of hardware that is suitable for this purpose. More specifically, temperature sensors 800 (e.g., thermistors) are positioned in thermal contact with respective electrode elements (for example, dielectric material 310 / layer of metal 320) within each of the electrode assemblies 50a. The temperature sensors 800 measure respective first and second temperatures (e.g., at first and second electrode elements in the first electrode assembly and second electrode assembly, respectively), and the controller 822 controls the output of the AC voltage generator 820 based on these temperatures.

Similar embodiments and methods are envisaged utilizing any of the electrode assemblies 50a-e, or combinations thereof, in place of either or both of the first electrode assembly 50a and the second electrode assembly 50a.

The electrode assemblies described herein may be used to apply an alternating electric field to a target region of a subject's body to treat a condition present in the subject. Such methods include positioning a first electrode assembly and a second electrode assembly on opposite sides of a target region of a subject's body, wherein the first and second electrode assemblies are as described herein, and applying an alternating electric field between the first and second electrode assemblies to treat a condition in the subject. The target region may be any region of the subject's body which is impaired by a condition or presents a condition. Suitable target regions include, but are not limited to, the brain, torso, abdomen, lung, breast, ovary, prostate, pancreas, stomach, intestine, colon, rectum, liver, or kidney of the subject. In some embodiments the target region may include a tumor or a cancer (one or more cancer cells).

By "treat" is meant to administer or apply a therapeutic, such as alternating electric fields, to a subject, such as a human or other mammal (for example, an animal model), that has a tumor (cancer) or has an increased susceptibility for developing cancer, in order to prevent or delay a worsening of the effects of the tumor (cancer), or to partially or fully reverse the effects of the tumor (cancer).

The alternating electric field used in the methods and/or embodiments described herein may have a frequency between 50 kHz to 2 MHz, 50 kHz and 1 MHz, 100 kHz and 1 MHz, 100 kHz and 500 kHz, 100 kHz and 300 kHz, or 150 kHz and 400 kHz. In some embodiments, the alternating electric field has a frequency of about 100 kHz, about 150 kHz, about 175 kHz, about 200 kHz, about 225 kHz, about 250 kHz, about 275 kHz, about 300 kHz, about 325 kHz, about 350 kHz, about 375 kHz, about 400 kHz, about 425 kHz, about 450 kHz, about 475 kHz, about 500 kHz, about 550 kHz, about 600 kHz, about 650 kHz, about 700 kHz, about 750 kHz, about 800 kHz, about 850 kHz, about 900 kHz, about 950 kHz, or about 1 MHz. The frequencies and frequency ranges listed above may be useful in any of the methods described herein.

In some methods and/or embodiments, the alternating electric field has an electric field strength between 0.1 V/cm and 20 V/cm, 0.1 V/cm and 10 V/cm, or 1 V/cm and 4 V/cm. The electric field strength ranges listed above may be useful in any of the methods and/or embodiments described herein.

Some methods and/or embodiments include positioning a first electrode assembly and a second electrode assembly on opposite sides of a target site in a subject's body, wherein the first and second electrode assemblies are as described herein, and applying an alternating electric field between the first and second electrode assemblies to treat a tumor or a cancer (region containing cancer cells) in the subject.

The methods and embodiments described herein may be used to treat a tumor or cancer in a subject. Such tumors (cancer) include, but are not limited to, brain cancers such as glioblastoma multiforme, lung cancers (e.g., malignant pleural mesothelioma, non-small cell lung cancer), brain metastases (e.g., from non-small cell lung cancer or melanoma), breast cancer, ovarian cancer, prostate cancer, pancreatic cancer, gastric cancer (e.g., gastroesophageal junction adenocarcinoma, gastric adenocarcinoma), intestinal cancer, colon cancer, rectal cancer, liver cancer, hepatocellular cancer, and uveal cancer (eye cancer).

The scope of the invention is defined by the appended claims.

## Claims

1. An apparatus for applying an alternating electric field to a subject's body, the apparatus comprising:
a sheet of anisotropic material (70) having a front face and a rear face, the front face being configured to face the subject's skin, wherein the sheet of anisotropic material (70) has a first thermal conductivity in a direction that is perpendicular to the front face thereof, and a thermal conductivity of the sheet of anisotropic material (70) in directions that are parallel to the front face thereof is more than two times higher than the first thermal conductivity;
at least one layer of conductive material (60) disposed in electrical contact with the front face of the sheet of anisotropic material (70), wherein the at least one layer of conductive material (60) has a biocompatible front surface; and
at least one electrode element (E1, E2) positioned behind the sheet of anisotropic material (70), wherein the at least one electrode element (E1, E2) has a front face disposed in electrical contact with the rear face of the sheet of anisotropic material (70).

2. The apparatus of claim 1, comprising a plurality of electrode elements (E1, E2), wherein each of the electrode elements (E1, E2) comprises a layer of dielectric material (310) having a front face and a rear face, the front face of the layer of dielectric material (310) being the front face of the electrode element (E1, E2), and a layer of metal (320) disposed on the rear face of the layer of dielectric material (310), and further comprising:
a rear layer of conductive material (80) positioned between the front faces of the electrode elements (E1, E2) and the rear face of the sheet of anisotropic material (70).

3. The apparatus of claim 1, wherein each of the at least one electrode element (E1, E2) comprises a layer of dielectric material (310) having a front face and a rear face, the front face of the layer of dielectric material (310) being the front face of the at least one electrode element (E1, E2), a layer of metal (320) disposed on the rear face of the layer of dielectric material (310), and a rear layer of conductive material (380) positioned between the front face of each of the at least one electrode element (E1, E2) and the rear face of the sheet of anisotropic material (70).

4. The apparatus of claim 2 or 3, wherein the rear layer of conductive material (80; 380) comprises conductive hydrogel.

5. The apparatus of claim 2 or 3, wherein the rear layer of conductive material (80; 380) comprises a conductive adhesive.

6. The apparatus of claim 5, wherein the conductive adhesive comprises an adhesive polymer and carbon powder, particles, fibers, flakes or nanotubes.

7. The apparatus of claim 5, wherein the conductive adhesive has a thickness between 10 and 2,000 µm.

8. The apparatus of claim 1, wherein the at least one electrode element (E1, E2) comprises a piece of metal (500; 600) having a front face, the front face of the piece of metal (500; 600) being the front face of the at least one electrode element (E1, E2).

9. The apparatus of claim 8, further comprising:
a rear layer of conductive material (80; 380) positioned between the front face of the at least one electrode element (E1, E2) and the rear face of the sheet of anisotropic material (70).

10. The apparatus of claim 8, wherein the front face of the at least one electrode element (E1, E2) is positioned in direct contact with the rear face of the sheet of anisotropic material (70).

11. The apparatus of claim 1, wherein the sheet of anisotropic material (70) is a sheet of graphite.

12. The apparatus of claim 1, wherein the sheet of anisotropic material (70) is a sheet of pyrolytic graphite, a graphite foil of compressed high-purity exfoliated mineral graphite or a graphitized polymer film.

13. The apparatus of claim 1, wherein the sheet of anisotropic material (70) is nonmetallic.

14. The apparatus of claim 1, wherein the thermal conductivity of the sheet of anisotropic material (70) in directions that are parallel to the front face thereof is more than ten times higher than the first thermal conductivity.

15. The apparatus of claim 1, wherein the sheet of anisotropic material (70) has a first resistance in a direction that is perpendicular to the front face thereof, and a resistance of the sheet of anisotropic material (70) in directions that are parallel to the front face thereof is less than half of the first resistance.

16. The apparatus of claim 1, wherein the at least one layer of conductive material (60) comprises hydrogel.

17. The apparatus of claim 1, wherein the at least one layer of conductive material (60) comprises a layer of hydrogel with a thickness between 50 and 2000 µm.

18. The apparatus of claim 1, wherein the at least one layer of conductive material (60) comprises a conductive adhesive.

19. The apparatus of claim 18, wherein the conductive adhesive comprises an adhesive polymer and carbon powder, particles, fibers, flakes or nanotubes.

20. The apparatus of claim 18, wherein the conductive adhesive has a thickness between 10 and 2,000 µm.

21. The apparatus of claim 1, further comprising:
a flexible, self-adhesive backing (55) configured to support the sheet of anisotropic material (70), the at least one electrode element (E1, E2) and the at least one layer of conductive material (60) so that the front surface of the at least one layer of conductive material (60) can be positioned against the subject's skin.

22. The apparatus of claim 1, wherein the centroid of the front face of the at least one electrode element (E1, E2) is positioned less than 3 cm away from the centroid of the sheet of anisotropic material (70).

23. The apparatus of claim 1, wherein the centroid of the front face of the at least one electrode element (E1, E2) is positioned away from the centroid of the sheet of anisotropic material (70) by less than 30% of a dimension in a direction parallel to the rear face of the sheet of anisotropic material (70).

24. The apparatus of claim 1, comprising a plurality of electrode elements (E1, E2).

25. The apparatus of claim 1, wherein the at least one layer of conductive material (60) is the same size or larger than the sheet of anisotropic material (70).

## Patentansprüche

1. Einrichtung zum Anlegen eines elektrischen Wechselfeldes an einen Körper eines Subjekts, wobei die Einrichtung Folgendes umfasst:
eine Lage aus anisotropem Material (70), die eine Vorder- und eine Rückseite aufweist, wobei die Vorderseite konfiguriert ist, der Haut des Subjekts zugewandt zu sein, wobei die Lage aus anisotropem Material (70) eine erste Wärmeleitfähigkeit in einer Richtung senkrecht zu ihrer Vorderseite aufweist und eine Wärmeleitfähigkeit der Lage aus anisotropem Material (70) in Richtungen parallel zu ihrer Vorderseite mehr als doppelt so hoch ist wie die erste Wärmeleitfähigkeit;
mindestens eine Schicht aus leitfähigem Material (60), die in elektrischem Kontakt mit der Vorderseite der Lage aus anisotropem Material (70) angeordnet ist, wobei die mindestens eine Schicht aus leitfähigem Material (60) eine biokompatible Vorderseite aufweist; und
mindestens ein Elektrodenelement (E1, E2), das hinter der Lage aus anisotropem Material (70) positioniert ist, wobei das mindestens eine Elektrodenelement (E1, E2) eine Vorderseite aufweist, die in elektrischem Kontakt mit der Rückseite der Lage aus anisotropem Material (70) angeordnet ist.

2. Einrichtung nach Anspruch 1, umfassend eine Vielzahl von Elektrodenelementen (E1, E2), wobei jedes der Elektrodenelemente (E1, E2) eine Schicht aus dielektrischem Material (310) umfasst, die eine Vorder- und eine Rückseite aufweist, wobei die Vorderseite der Schicht aus dielektrischem Material (310) die Vorderseite des Elektrodenelements (E1, E2) bildet, und eine Metallschicht (320), die auf der Rückseite der Schicht aus dielektrischem Material (310) angeordnet ist, weiter umfassend:
eine rückseitige Schicht aus leitfähigem Material (80), die zwischen den Vorderseiten der Elektrodenelemente (E1, E2) und der Rückseite der Lage aus anisotropem Material (70) positioniert ist.

3. Einrichtung nach Anspruch 1, wobei jedes der mindestens einen Elektrodenelemente (E1, E2) eine Schicht aus dielektrischem Material (310), die eine Vorder- und eine Rückseite aufweist, wobei die Vorderseite der Schicht aus dielektrischem Material (310) die Vorderseite des mindestens einen Elektrodenelements (E1, E2) bildet, eine Metallschicht (320), die auf der Rückseite der Schicht aus dielektrischem Material (310) angeordnet ist, und eine rückseitige Schicht aus leitfähigem Material (380), die zwischen der Vorderseite jedes der mindestens einen Elektrodenelemente (E1, E2) und der Rückseite der Lage aus anisotropem Material (70) positioniert ist, umfasst.

4. Einrichtung nach Anspruch 2 oder 3, wobei die rückseitige Schicht aus leitfähigem Material (80; 380) leitfähiges Hydrogel umfasst.

5. Einrichtung nach Anspruch 2 oder 3, wobei die rückseitige Schicht aus leitfähigem Material (80; 380) einen leitfähigen Klebstoff umfasst.

6. Einrichtung nach Anspruch 5, wobei der leitfähige Klebstoff ein Klebstoffpolymer und Kohlenstoffpulver, -partikel, -fasern, -flocken oder -nanoröhren umfasst.

7. Einrichtung nach Anspruch 5, wobei der leitfähige Klebstoff eine Dicke zwischen 10 und 2.000 µm aufweist.

8. Einrichtung nach Anspruch 1, wobei das mindestens eine Elektrodenelement (E1, E2) ein Metallstück (500; 600), die eine Vorderseite, wobei die Vorderseite des Metallstücks (500; 600) die Vorderseite des mindestens einen Elektrodenelements (E1, E2) bildet.

9. Einrichtung nach Anspruch 8, weiter umfassend:
eine rückseitige Schicht aus leitfähigem Material (80; 380), die zwischen der Vorderseite des mindestens einen Elektrodenelements (E1, E2) und der Rückseite der Lage aus anisotropem Material (70) positioniert ist.

10. Einrichtung nach Anspruch 8, wobei die Vorderseite des mindestens einen Elektrodenelements (E1, E2) in direktem Kontakt mit der Rückseite der Lage aus anisotropem Material (70) positioniert ist.

11. Einrichtung nach Anspruch 1, wobei es sich bei der Lage aus anisotropem Material (70) um eine Lage aus Graphit handelt.

12. Einrichtung nach Anspruch 1, wobei es sich bei der Lage aus anisotropem Material (70) um eine Lage aus pyrolytischem Graphit, eine Graphitfolie aus komprimiertem, hochreinem, exfoliertem Mineralgraphit oder einen graphitisierten Polymerfilm handelt.

13. Einrichtung nach Anspruch 1, wobei die Lage aus anisotropem Material (70) nichtmetallisch ist.

14. Einrichtung nach Anspruch 1, wobei die Wärmeleitfähigkeit der Lage aus anisotropem Material (70) in Richtungen parallel zu ihrer Vorderseite mehr als zehnmal höher ist als die erste Wärmeleitfähigkeit.

15. Einrichtung nach Anspruch 1, wobei die Lage aus anisotropem Material (70) einen ersten Widerstand in einer Richtung aufweist, die senkrecht zu ihrer Vorderseite verläuft, und ein Widerstand der Lage aus anisotropem Material (70) in Richtungen, die parallel zu ihrer Vorderseite verlaufen, weniger als die Hälfte des ersten Widerstands beträgt.

16. Einrichtung nach Anspruch 1, wobei die mindestens eine Schicht aus leitfähigem Material (60) Hydrogel umfasst.

17. Einrichtung nach Anspruch 1, wobei die mindestens eine Schicht aus leitfähigem Material (60) eine Hydrogelschicht mit einer Dicke zwischen 50 und 2000 µm umfasst.

18. Einrichtung nach Anspruch 1, wobei die mindestens eine Schicht aus leitfähigem Material (60) einen leitfähigen Klebstoff umfasst.

19. Einrichtung nach Anspruch 18, wobei der leitfähige Klebstoff ein Klebstoffpolymer und Kohlenstoffpulver, -partikel, -fasern, -flocken oder -nanoröhren umfasst.

20. Einrichtung nach Anspruch 18, wobei der leitfähige Klebstoff eine Dicke zwischen 10 und 2.000 µm aufweist.

21. Einrichtung nach Anspruch 1, weiter umfassend:
eine flexible, selbstklebende Unterlage (55), die so konfiguriert ist, dass sie die Lage aus anisotropem Material (70), das mindestens eine Elektrodenelement (E1, E2) und die mindestens eine Schicht aus leitfähigem Material (60) trägt, sodass die Vorderseite der mindestens einen Schicht aus leitfähigem Material (60) an der Haut des Subjekts positioniert werden kann.

22. Einrichtung nach Anspruch 1, wobei der Schwerpunkt der Vorderseite des mindestens einen Elektrodenelements (E1, E2) weniger als 3 cm vom Schwerpunkt der Lage aus anisotropem Material (70) entfernt positioniert ist.

23. Einrichtung nach Anspruch 1, wobei der Schwerpunkt der Vorderseite des mindestens einen Elektrodenelements (E1, E2) vom Schwerpunkt der Lage aus anisotropem Material (70) um weniger als 30 % einer Abmessung in einer Richtung parallel zur Rückseite der Lage aus anisotropem Material (70) entfernt positioniert ist.

24. Einrichtung nach Anspruch 1, umfassend eine Vielzahl von Elektrodenelementen (E1, E2).

25. Einrichtung nach Anspruch 1, wobei die mindestens eine Schicht aus leitfähigem Material (60) gleich groß wie oder größer als die Lage aus anisotropem Material (70) ist.

## Revendications

1. Appareil destiné à appliquer un champ électrique alternatif au corps d'un sujet, l'appareil comprenant :
une feuille de matériau anisotrope (70) présentant une face avant et une face arrière, la face avant étant configurée pour faire face à la peau du sujet, dans lequel la feuille de matériau anisotrope (70) présente une première conductivité thermique dans une direction perpendiculaire à sa face avant, et une conductivité thermique de la feuille de matériau anisotrope (70) dans des directions parallèles à sa face avant est plus de deux fois supérieure à la première conductivité thermique ;
au moins une couche de matériau conducteur (60) disposée en contact électrique avec la face avant de la feuille de matériau anisotrope (70), dans lequel la au moins une couche de matériau conducteur (60) présente une surface avant biocompatible ; et
au moins un élément d'électrode (E1, E2) positionné derrière la feuille de matériau anisotrope (70), dans lequel le au moins un élément d'électrode (E1, E2) présente une face avant disposée en contact électrique avec la face arrière de la feuille de matériau anisotrope (70).

2. Appareil selon la revendication 1, comprenant une pluralité d'éléments d'électrode (E1, E2), dans lequel chacun des éléments d'électrode (E1, E2) comprend une couche de matériau diélectrique (310) présentant une face avant et une face arrière, la face avant de la couche de matériau diélectrique (310) étant la face avant de l'élément d'électrode (E1, E2), et une couche de métal (320) disposée sur la face arrière de la couche de matériau diélectrique (310), et comprenant en outre :
une couche arrière de matériau conducteur (80) positionnée entre les faces avant des éléments d'électrode (E1, E2) et la face arrière de la feuille de matériau anisotrope (70).

3. Appareil selon la revendication 1, dans lequel chacun des au moins un élément d'électrode (E1, E2) comprend une couche de matériau diélectrique (310) présentant une face avant et une face arrière, la face avant de la couche de matériau diélectrique (310) étant la face avant du au moins un élément d'électrode (E1, E2), une couche de métal (320) disposée sur la face arrière de la couche de matériau diélectrique (310), et une couche arrière de matériau conducteur (380) positionnée entre la face avant de chacun du au moins un élément d'électrode (E1, E2) et la face arrière de la feuille de matériau anisotrope (70).

4. Appareil selon la revendication 2 ou 3, dans lequel la couche arrière de matériau conducteur (80 ; 380) comprend un hydrogel conducteur.

5. Appareil selon la revendication 2 ou 3, dans lequel la couche arrière de matériau conducteur (80 ; 380) comprend un adhésif conducteur.

6. Appareil selon la revendication 5, dans lequel l'adhésif conducteur comprend un polymère adhésif et de la poudre de carbone, des particules, des fibres, des flocons ou des nanotubes.

7. Appareil selon la revendication 5, dans lequel l'adhésif conducteur présente une épaisseur comprise entre 10 et 2000 µm.

8. Appareil selon la revendication 1, dans lequel le au moins un élément d'électrode (E1, E2) comprend une pièce de métal (500 ; 600) présentant une face avant, la face avant de la pièce de métal (500 ; 600) étant la face avant du au moins un élément d'électrode (E1, E2).

9. Appareil selon la revendication 8, comprenant en outre :
une couche arrière de matériau conducteur (80 ; 380) positionnée entre la face avant du au moins un élément d'électrode (E1, E2) et la face arrière de la feuille de matériau anisotrope (70).

10. Appareil selon la revendication 8, dans lequel la face avant du au moins un élément d'électrode (E1, E2) est positionnée en contact direct avec la face arrière de la feuille de matériau anisotrope (70).

11. Appareil selon la revendication 1, dans lequel la feuille de matériau anisotrope (70) est une feuille de graphite.

12. Appareil selon la revendication 1, dans lequel la feuille de matériau anisotrope (70) est une feuille de graphite pyrolytique, une feuille de graphite de graphite minéral exfolié compressé de haute pureté ou un film polymère graphitisé.

13. Appareil selon la revendication 1, dans lequel la feuille de matériau anisotrope (70) est non métallique.

14. Appareil selon la revendication 1, dans lequel la conductivité thermique de la feuille de matériau anisotrope (70) dans les directions parallèles à sa face avant est plus de dix fois supérieure à la première conductivité thermique.

15. Appareil selon la revendication 1, dans lequel la feuille de matériau anisotrope (70) présente une première résistance dans une direction perpendiculaire à sa face avant, et une résistance de la feuille de matériau anisotrope (70) dans des directions parallèles à sa face avant est inférieure à la moitié de la première résistance.

16. Appareil selon la revendication 1, dans lequel la au moins une couche de matériau conducteur (60) comprend un hydrogel.

17. Appareil selon la revendication 1, dans lequel la au moins une couche de matériau conducteur (60) comprend une couche d'hydrogel d'une épaisseur comprise entre 50 et 2000 µm.

18. Appareil selon la revendication 1, dans lequel la au moins une couche de matériau conducteur (60) comprend un adhésif conducteur.

19. Appareil selon la revendication 18, dans lequel l'adhésif conducteur comprend un polymère adhésif et de la poudre de carbone, des particules, des fibres, des flocons ou des nanotubes.

20. Appareil selon la revendication 18, dans lequel l'adhésif conducteur présente une épaisseur comprise entre 10 et 2000 µm.

21. Appareil selon la revendication 1, comprenant en outre :
un support flexible et auto-adhésif (55) configuré pour supporter la feuille de matériau anisotrope (70), le au moins un élément d'électrode (E1, E2) et la au moins une couche de matériau conducteur (60) de sorte que la surface avant de la au moins une couche de matériau conducteur (60) peut être positionnée contre la peau du sujet.

22. Appareil selon la revendication 1, dans lequel le centre de gravité de la face avant du au moins un élément d'électrode (E1, E2) est positionné à moins de 3 cm du centre de gravité de la feuille de matériau anisotrope (70).

23. Appareil selon la revendication 1, dans lequel le centre de gravité de la face avant du au moins un élément d'électrode (E1, E2) est positionné à moins de 30 % d'une dimension du centre de gravité de la feuille de matériau anisotrope (70) dans une direction parallèle à la face arrière de la feuille de matériau anisotrope (70).

24. Appareil selon la revendication 1, comprenant une pluralité d'éléments d'électrode (E1, E2).

25. Appareil selon la revendication 1, dans lequel la au moins une couche de matériau conducteur (60) est de la même taille ou plus grande que la feuille de matériau anisotrope (70).
